# FASCICULE DE BREVET EUROPEEN

(11) **EP 3 595 617 B1**
(45) Date de publication et mention de la délivrance du brevet: **30.06.2021**
(21) Numéro de dépôt: 18708698.8
(22) Date de dépôt: 07.03.2018
(51) Int. Cl.: A61K 8/25, A61K 8/37, A61Q 3/02, A61K 8/85, A61K 8/19

(54) **COMPOSITIONS POUR VERNIS A ONGLES**
NAGELZUSAMMENSETZUNG
NAIL ENAMEL COMPOSITION

(30) Priorité: 13.03.2017 FR 1752039
(43) Date de publication de la demande: 22.01.2020
(73) Titulaire: Fiabila SAS, 28130 Maintenon (FR)
(72) Inventeur: MARTINEZ, Francisco, 28000 Chartres (FR)
(74) Mandataire: Hirsch & Partners
(86) Numéro de dépôt international: PCT/EP2018/055655
(87) Numéro de publication internationale: WO 2018/166874

(56) Documents cités:
- EP-A1- 0 714 653
- EP-A1- 0 819 420
- US-A1- 2001 007 676

## Description

### DOMAINE DE L'INVENTION

La présente invention concerne de nouvelles compositions cosmétiques nitrocellulosiques à propriétés améliorées qui sont plus particulièrement utilisables comme, ou pour la préparation de, vernis à ongles, lesdites compositions étant parfaitement stables tout en étant exemptes de composés argileux, en particulier d'argiles modifiées.

### ARRIERE-PLAN TECHNIQUE

Les vernis à ongles ont peu changé depuis leur conception initiale. Ils peuvent être utilisés non seulement pour habiller et embellir les ongles (maquillage) mais également pour les protéger (soin). Dans le domaine considéré, les ongles s'entendent évidemment tant des ongles des mains que des ongles des pieds.

Pour la formulation d'un vernis à ongle classique, on fait appel à plusieurs ingrédients conventionnels, parmi lesquels figurent :
- un agent filmogène principal, cet agent apportant un séchage rapide, l'adhésion sur l'ongle, de la dureté et de la brillance. Il doit toutefois être plastifié car il est cassant. De nos jours, la nitrocellulose (encore appelée communément «nitrate de cellulose») est de loin l'agent filmogène principal le plus utilisé dans les vernis à ongles. Cette substance présente en effet de nombreuses propriétés compatibles avec l'application qui en est faite : la transparence, l'adhérence et le séchage, faisant ainsi d'elle une matière première polyvalente, incontournable et presque universelle dans le monde des vernis à ongles commerciaux.
- une ou plusieurs résines secondaires (polymères) destinées à améliorer la qualité du film : l'agent filmogène principal est complété dans les formulations commerciales par une ou des résines texturantes permettant d'augmenter l'extrait sec et donc de contrôler la quantité de film formé à la surface de l'ongle, et ainsi augmenter certaines propriétés comme le brillant ou l'adhérence. Pendant longtemps, les résines secondaires utilisées étaient des produits obtenus par condensation du toluène sulfonamide et du formaldéhyde, et connus notamment sous le nom commercial de Santolite®. Ces derniers produits, pour des raisons de sécurité (taux de formol résiduel élevé), sont toutefois de plus en plus abandonnés pour être remplacés par d'autres types de résines, comme par exemple des résines polyester, considérées comme plus acceptables d'un point de vue cosmétique.
- un ou plusieurs plastifiants de l'agent filmogène principal pour optimiser et modifier la flexibilité et la souplesse du film.
- un ou plusieurs solvants usuels : ces solvants, de nature le plus souvent organique (acétone, acétate d'éthyle ou acétate de butyle notamment) sont mélangés aux autres ingrédients pour former une composition plus ou moins fluide facilement applicable sur l'ongle avec un pinceau. En particulier, ils doivent être capables de dissoudre l'agent filmogène et les résines secondaires. Ils permettent également d'ajuster la viscosité du vernis à une valeur adaptée. Enfin, la vitesse d'évaporation du solvant (solvant volatile) ne doit être ni trop rapide ni trop lente pour obtenir le meilleur compromis brillance/séchage.
- une ou plusieurs matières colorantes : les colorants solubles dans le support de formulation ne sont pratiquement jamais utilisés dans le domaine des vernis à ongles. Par contre, on fait appel de façon très fréquente voire systématique à des pigments (insolubles dans le milieu de formulation), organiques et/ou minéraux, autorisés par la réglementation dans des applications cosmétiques. Il est connu que les pigments minéraux ont tendance à favoriser le phénomène de sédimentation. Les pigments organiques, quant à eux, sont plus sensibles aux phénomènes de stabilité chimique. La composition peut aussi contenir, comme ou en complément des pigments, des nacres et/ou paillettes de différentes natures.

Les compositions de vernis à ongles préparées à partir des ingrédients ci-dessus sont, pour la vente, conditionnées dans des récipients ou flacons, généralement fermés de façon hermétique au moyen de bouchons intégrant des pinceaux destinés à l'application du produit sur l'ongle.

Cette présentation et cette utilisation impliquent deux contraintes particulières aux formulateurs.

La première difficulté est qu'il convient de limiter au maximum, en situation de stockage et de repos du produit, la sédimentation potentielle des pigments et/ou nacres au sein de la composition afin de maintenir son homogénéité dans le flacon. Il convient également d'éviter l'apparition de phénomènes de synérèse. Pour aider à lutter contre ces phénomènes de sédimentation et de stabilité physique, on fait souvent appel à des colloïdes stabilisants. Ces colloïdes sont le plus souvent des argiles organophiles (argiles modifiées), obtenues par substitution des cations minéraux par des cations organiques à partir de qualité d'argiles courantes, telles que bentonites, hectorites, saponites ou montmorillonites.

La deuxième difficulté réside dans le fait que, après son prélèvement dans le flacon par un utilisateur, le vernis à ongle doit pouvoir s'étaler facilement sur l'ongle mais ne plus couler après application, ce qui implique que le vernis puisse retrouver sa viscosité initiale dès l'instant où il n'est plus mis en mouvement avec le pinceau.

Comme indiqué précédemment, pour obtenir ces propriétés de stabilité et de facilité d'étalement, on fait très souvent appel à des agents rhéologiques de type argileux, et plus particulièrement à des argiles modifiées. Toutefois, l'emploi de ces agents n'est pas exempt d'inconvénients. En effet, les argiles organophiles sont des argiles modifiées avec des composés chimiques capables de rendre l'argile apte à gonfler dans les milieux solvants, et ces composés chimiques sont souvent choisis parmi les amines quaternaires, les amines tertiaires, les acétates aminés, les imidazolines, les savons aminés ou les oxides aminés, pris seuls ou en mélanges. A ce titre, on peut citer les quaternium-18 bentonites telles que celles vendues sous les dénomination Bentone 3, Bentone 38, Bentone 38 V par la Société Elementis, Tixogel VP par la Société Eckart, Claytone 34, Claytone 40, Clayton XL par la Société Eckart; les stéaralkonium bentonites et hectorites telles que celles vendues sous les dénominations commerciales Bentone 27V par la Société Elementis, Tixogel LG ou MP Z par la Société Eckart, Claytone AF, Claytone APA par la Société Eckart; les quaternium-18 /benzalkonium bentonites telles que celles vendues sous les dénominations Claytone HT, Claytone PS par la Société Eckart, cette liste n'étant nullement limitative. Or, ces composés à fonction amine peuvent induire dans la composition finale des propriétés indésirables, notamment en terme de sécurité, en particulier en réagissant lors de leur mise en contact avec d'autres ingrédients présents dans la composition, comme par exemple la nitrocellulose et/ou des pigments, ce qui peut générer des espèces néfastes à une bonne utilisation comme vernis à ongles.

Pour ces différentes raisons, de nombreuses tentatives ont été faites pour essayer de substituer les argiles modifiées par d'autres systèmes, même si, aujourd'hui encore, ces argiles modifiées, comme rappelé précédemment, continue d'être utilisées de façon quasi-universelle dans la préparation des vernis à ongles commerciaux.

Dans le document US 2001/0007676, on a ainsi proposé de remplacer les argiles organomodifiées par de la silice pyrogénée. Toutefois, les résultats obtenus ne sont pas complètement satisfaisants, ceci pouvant s'expliquer par le fait que d'une part les caractéristiques de la silice pyrogénée ne sont pas précisées et que, d'autre part, la nature des résines secondaires à employer à côté de la nitrocellulose n'a pas été convenablement identifiée.

La présente invention vise à résoudre, pour partie ou en totalité, les problèmes des compositions de vernis à ongles de l'art antérieur à base de nitrocellulose, qu'il s'agisse de celles contenant des composés argileux de type argile modifiée ou encore de celles qui en sont dépourvues.

Plus précisément, l'invention vise à proposer des compositions pour vernis à ongles, exemptes ou substantiellement exemptes de composés argileux, qui soient stables physiquement et chimiquement, et qui présentent d'excellentes propriétés d'usage en application, notamment d'adhérence et de tenue sur l'ongle, ainsi qu'un excellent profil en terme de sécurité et de respect de la réglementation.

A la suite d'importantes recherches menées sur cette question, il a maintenant été trouvé que cet objectif, et d'autres, pouvaient être atteints au moyen d'une formulation particulière à base de nitrocellulose contenant une association unique d'ingrédients, à savoir une association entre une silice pyrogénée à haute surface spécifique et une résine contenant des motifs aromatiques, qui permette de répondre de façon acceptable à l'ensemble des propriétés requises pour un vernis à ongles destiné à un usage commercial, tout en s'affranchissant de l'emploi de composés argileux, modifiés ou non.

### RESUME DE L'INVENTION

L'invention a ainsi pour premier objet une composition pour vernis à ongles comprenant, dans un ou des solvants organiques cosmétiquement acceptables :
- un agent filmogène principal comprenant de la nitrocellulose,
- au moins une silice pyrogénée présentant une surface spécifique BET d'au moins 170m²/g,
- au moins une résine secondaire présentant dans sa structure chimique au moins un groupement aromatique,
- au moins un plastifiant,
- au moins une matière colorante, insoluble dans ledit solvant,
ladite composition étant exempte ou substantiellement exempte de composés argileux, notamment d'argiles modifiées, en particulier d'argiles organophiles.

Selon un mode de réalisation, la composition est exempte de tout composé argileux, notamment d'argiles modifiées, en particulier des argiles organophiles.

Selon un autre mode de réalisation, les matières colorantes sont des pigments organiques ou minéraux.

Selon un autre mode de réalisation, la silice pyrogénée est une silice pyrogénée de type hydrophile ou de type hydrophobe.

Selon un autre mode de réalisation, la composition selon l'invention est non aqueuse.

Selon un autre mode de réalisation, la composition selon l'invention comprend au moins 10% en poids de nitrocellulose par rapport au poids total de la composition.

Selon un mode particulièrement préféré de réalisation, la composition pour vernis à ongles selon l'invention est anhydre, exempte de composés argileux du type argiles modifiées, et comprend, ou de préférence consiste en :
- un ou plusieurs solvants organiques cosmétiquement acceptables à hauteur de 10% à 95%, de préférence de 30% à 90%, et plus préférentiellement encore de 50% à 85%,
- un agent filmogène principal comprenant de la nitrocellulose à hauteur de 5% à 30%, de préférence de 5% à 25%, encore plus préférentiellement de 10% à 22%,
- au moins une silice pyrogénée de surface spécifique supérieure ou égale à 170 m²/g à hauteur de 0.1% à 6%, de préférence de 0.3% à 5%, plus préférentiellement encore de 0.5% à 4%,
- au moins une résine secondaire possédant dans sa structure au moins un groupement (motif) aromatique, ladite résine étant présente à hauteur de 1% à 20%, de préférence de 2% à 20%, plus préférentiellement encore de 3% à 15%, ces concentrations étant exprimées en extrait sec de résine,
- au moins un plastifiant à hauteur de 1% à 15 %, de préférence de 2% à 12%, plus préférentiellement encore de 4% à 10%,
- au moins une matière colorante, de préférence un pigment, à hauteur de 0.001% à 15%, de préférence de 0.005% à 12%, plus préférentiellement encore de 0.01% à 10%,
les pourcentages ci-dessus étant exprimés en poids par rapport à l'ensemble de la composition.

Les avantages de l'invention sont multiples : les vernis à ongles peuvent être totalement exempts de composés argileux, en particulier d'argiles modifiées ; ils sont parfaitement stables chimiquement (absence de dégradation et/ou de changement de couleur) et physiquement (absence de sédimentation et/ou de synérèse) ; ils sont d'une application extrêmement aisée ; et les films obtenus sur l'ongle après séchage présentent d'excellentes qualités.

Un second objet porte sur l'utilisation des compositions selon l'invention pour la protection et/ou le maquillage des ongles.

Un troisième objet concerne un procédé de protection et/ou de maquillage des ongles, qui consiste essentiellement à appliquer sur ces derniers une composition selon l'invention.

Un dernier objet, enfin, concerne des articles conditionnés, prêts à l'emploi, contenant la composition selon l'invention.

### DESCRIPTION DE MODES DE REALISATION DE L'INVENTION

L'invention va maintenant être décrite plus en détail et de façon non limitative dans les divers aspects qui la composent.

### Solvant organique

La composition pour vernis à ongles selon l'invention comprend au moins un solvant organique, permettant de solubiliser les substances polymériques qu'elle contient. Le système solvant peut être constitué d'un seul solvant ou d'un mélange de solvants. Ce solvant peut ainsi être choisi parmi :
- les cétones liquides à température ambiante tels que la méthyléthyl cétone, l'acétone, la méthylisobutyl cétone ;
- les alcools liquides à température ambiante tels que l'éthanol, le propanol, le butanol, l'isopropanol, le diacétone d'alcool ;
- les esters à chaîne courte ayant de 3 à 8 atomes de carbone tels que l'acétate d'éthyle, l'acétate de propyle, l'acétate de butyle ;
- les alcanes liquides à température ambiante tels que l'heptane, le dodécane, l'hexane ;
- et leurs mélanges.

De préférence, le milieu solvant est anhydre ou substantiellement anhydre, c'est-à-dire que la composition se présente au final sous une forme non aqueuse, ou substantiellement non aqueuse, c'est-à-dire avec une teneur en poids d'eau inférieure à 1%.

Le solvant organique ou mélange de solvants est de préférence présent dans la composition dans une quantité comprise entre 10% et 95% en poids, de préférence entre 30% et 90% en poids, et plus préférentiellement encore entre 50% et 85% en poids, par rapport au poids total de la composition.

### Agent filmogène principal

La composition pour vernis à ongles selon l'invention comprend un agent filmogène principal qui comprend de la nitrocellulose. De préférence, cet agent filmogène principal est essentiellement constitué de nitrocellulose, et encore plus préférentiellement est uniquement constitué de nitrocellulose.

Les nitrocelluloses sont des produits bien connus en soi dans le domaine de la cosmétique, et qui sont par ailleurs largement disponibles dans le commerce auprès de nombreux fournisseurs.

La nitrocellulose présente dans la composition selon l'invention peut être en particulier choisie parmi la nitrocellulose DHX 8/13, DHX 20/35 et DHX 30/50, notamment commercialisées par la Société Nobel.

D'autres exemples de nitrocelluloses utilisables dans le cadre de la présente invention et disponibles commercialement sont notamment les nitrocelluloses RS ½ sec et RS ¼ sec de la Société KCNC.

Selon un mode de réalisation, la composition ne contient aucun agent filmogène principal autre que de la nitrocellulose.

La quantité d'agent filmogène principal constitué de nitrocellulose dans la composition pour vernis à ongles selon l'invention peut être de 5% à 30% en poids, de préférence de 5% à 25%, plus préférentiellement de 10% à 22%, par rapport au poids total de la composition.

### Résine secondaire

La composition pour vernis à ongles selon l'invention comprend en outre au moins une résine secondaire, différente de l'agent filmogène principal. Par résine secondaire, on entend un ou plusieurs polymère(s) en moindre proportion pondérale que l'agent filmogène principal comprenant la nitrocellulose.

Cette résine secondaire, qui peut avoir ou non des propriétés filmogènes, peut être choisie parmi les résines styrène acryliques, les résines polyesters, les résines alkydes, les résines polyuréthanes, les résines cétoniques, les résines époxy tosylamide, les résines époxy, les résines issues des produits de condensation d'aldéhydes tels que les résines arylsulfonamide formaldéhyde comme la résine toluène sulfonamide formaldéhyde, et leurs mélanges. L'utilisation de ces résines, qui peuvent être synthétiques (de type radicalaire ou de type polycondensat) ou encore d'origine naturelle, est bien connue dans le domaine de la cosmétique, en particulier celui des vernis à ongles.

Selon une caractéristique absolument essentielle de la présente invention, seuls les polymères présentant dans leur structure chimique des groupements (ou motifs) aromatiques, monocycliques ou polycycliques, mais de préférence monocycliques (de type benzène), doivent être mis en œuvre.

De préférence, le ou les groupements aromatiques sont présents dans (à l'intérieur de) la chaine principale du polymère filmogène, mais il n'est pas exclu que ces groupements soient également portés de façon latérale (sous forme de radicaux) sur cette chaine.

Pour un même polymère, on préfère que tous les groupements aromatiques soient de nature identique, bien que le cas de polymères avec des groupements aromatiques de nature différente soit également envisageable.

Les polymères précités peuvent être utilisés seuls ou en mélanges entre eux. Les polymères préférés selon l'invention sont sélectionnés dans la classe des résines polyester ou des résines époxy.

A titre d'exemple de produits utilisables dans le cadre de la présente invention, on peut citer les résines commercialement disponibles suivantes: Beckosol® OD 230-70-E de la société Dainippon (résine alkyde), Variplus® SK de la société Tego (résine cétonique), Polytex® E 75 de la Société Estron Chemical (résine époxy tosylamide), Uniplex 670 P de la Société Lanxess (résine polyester) et 70KC A de la Société KCI (résine polyester).

La quantité de résine(s) secondaire(s) dans la composition pour vernis à ongles selon l'invention peut aller de 1% à 20% en poids, de préférence de 2% à 20%, plus préférentiellement encore de 3% à 15%, par rapport au poids total de la composition, ces concentrations étant exprimées en extrait sec de résine.

### Plastifiant

La composition pour vernis à ongles selon l'invention comprend également au moins un plastifiant.

De façon connue dans le domaine, la fonction du plastifiant est de permettre d'ajuster le compromis dureté/flexibilité du film.

Selon l'invention le plastifiant peut ainsi être choisi, seul ou en mélange, parmi :
- les esters d'acides, notamment carboxyliques, tels que les citrates, les benzoates, les adipates et les carbonates ;
- les diesters d'isosorbide.

L'isosorbide est un produit obtenu par déshydratation d'un dérivé du glucose, le sorbitol, qui peut être extrait de baies de sorbier ou de céréales. Le diester est avantageusement produit par réaction entre un acide gras d'origine végétale et l'isosorbide.

Parmi les ester de citrates, on peut citer à titre d'exemple, le triéthyl citrate, le tributyl citrate, le tributyl acétylcitrate. Et, parmi les esters de benzoates, on peut citer à titre d'exemple, le triméthyl pentanediol dibenzoate.

Le ou les plastifiants sont avantageusement présents dans la composition pour vernis à ongles dans une concentration comprise entre 1% et 15% en poids, de préférence entre 2% et 12%, plus préférentiellement encore entre 4% et 10%, en poids par rapport au poids total de la composition.

### Silice pyrogénée

Selon une caractéristique essentielle, la composition pour vernis à ongles selon l'invention comprend de la silice pyrogénée, et selon une autre caractéristique essentielle, ces silices pyrogénées doivent présenter une surface spécifique élevée, à savoir supérieure ou égale à 170 m²/g.

Par surface spécifique, on entend la surface spécifique BET, telle que mesurée selon méthode faisant l'objet de la norme ISO 9277.

Selon un mode de réalisation, la silice pyrogénée est choisie parmi les silices pyrogénées hydrophiles et les silices pyrogénées hydrophobes, et leurs mélanges.

De préférence, on utilise des silices pyrogénées présentant un caractère hydrophobe.

Les silices pyrogénées (hydrophiles et/ou hydrophobes) utilisées selon l'invention présentent de préférence des surfaces spécifiques (mesurée selon la méthode BET susmentionnée) supérieures ou égales à 190 m²/g, encore plus préférentiellement supérieures ou égales à 250 m²/g, et encore plus préférentiellement supérieures ou égales à 300 m²/g. Les silices pyrogénées présentant une surface spécifique allant de 250 à 350 m2/g conviennent particulièrement bien.

Selon l'invention, il est bien entendu possible d'utiliser des mélanges de silices pyrogénées différentes, dans la mesure où ces silices ont pour point commun de présenter de hautes surfaces spécifiques, comme indiqué précédemment.

Les silices pyrogénées hydrophiles peuvent être obtenues par hydrolyse à haute température d'un composé volatil du silicium dans une flamme oxhydrique, produisant une silice finement divisée. Les silices hydrophiles présentent un nombre important de groupements silanol à leurs surfaces. De telles silices hydrophiles sont par exemple commercialisées sous les dénominations "AEROSIL® 200", « AEROSIL® 255 », "AEROSIL® 300" et "AEROSIL® 380" par la Société Evonik, ou encore sous les dénominations CAB-O-SIL M-5 et CAB-O-SIL HS-5 par la Société Cabot, ou encore sous les dénominations HDK T30 et HDK N20 de la Société Wacker.

Les silices pyrogénées hydrophobes peuvent être obtenues par modification de la surface de la silice par une réaction chimique générant une diminution du nombre de groupes silanols, ces groupes pouvant être notamment substitués par des groupements hydrophobes. Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (14ème édition, 2012). Elles sont par exemple commercialisées sous la référence "AEROSIL® R812" par la Société Evonik, et sous la référence CAB-O-SIL TS 530 par la Société Cabot
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (14ème édition, 2012). Elles sont par exemple commercialisées sous la référence CAB-O-SIL TS 622 par la Société Cabot, ou encore sous la référence HDK H30 de la Société Wacker.

La silice pyrogénée à haute surface spécifique peut être présente dans la composition pour vernis à ongles en une teneur comprise entre 0.1 % et 6% en poids, de préférence entre 0.3% et 5%, plus préférentiellement encore entre 0.5% et 4%, en poids par rapport au poids total de la composition.

Selon une caractéristique importante de la présente invention, la composition est exempte, ou substantiellement exempte, de composés argileux. Par « substantiellement », on entend une concentration totale en composés argileux inférieure à, ou n'excédant pas, 5 000 ppm, de préférence inférieure à 1 000 ppm, plus préférentiellement encore inférieure à 500 ppm, et encore plus préférentiellement inférieure à 100 ppm. De préférence encore, la composition selon l'invention est totalement exempte de composé(s) argileux.

L'exclusion visée ci-dessus par l'invention au niveau de la présence des composés argileux concerne les argiles hydrophiles ou les argiles organophiles.

De façon connue, les argiles sont des silicates contenant un cation pouvant être choisi parmi les cations de calcium, de magnésium, d'aluminium, de sodium, de potassium, de lithium et leurs mélanges.

On entend par argile hydrophile une argile apte à gonfler dans l'eau, de manière à former après hydratation une dispersion colloidale. A titre d'exemples de tels produits, on peut citer les montmorillonites, les hectorites, les bentonites, les beidellites, les saponites ou les laponites ces argiles pouvant être d'origine naturelle ou synthétique.

Les argiles organophiles sont des argiles modifiées chimiquement (montmorillonites, bentonites, hectorites, atapulgites, sépiolites, mais de préférence une bentonite ou une hectorite) par certains composés de façon à rendre l'argile apte à gonfler dans les milieux solvants, ces composés étant généralement choisis parmi les amines quaternaires, les amines tertiaires, les acétates aminés, les imidazolines, les savons aminés, les sulfates gras, les alkyl aryl sulfonates, les oxides aminés, et leurs mélanges. A titre d'exemples typiques de tels argiles organophiles, on peut citer les quaternium-18 bentonites, les stéaralkonium bentonites et les quaternium-18/benzalkonium bentonites, les stéaralkonium hectorites, disponibles sous de nombreuses références commerciales.

### Matière(s) colorante(s)

La composition pour vernis à ongles selon l'invention comprend en outre au moins une matière colorante choisie par exemple parmi les pigments, les nacres et les paillettes.

Par pigments, il faut comprendre des particules de toute forme, blanches ou colorées, minérales ou organiques, naturelles ou synthétiques, insolubles dans le milieu de formulation (solvant), et destinées à apporter de la couleur à la composition.

Par nacres, il faut comprendre des particules de toute forme irisées, notamment produites par certains mollusques dans leur coquille ou bien synthétisées. Les principaux substrats des nacres sont les suivants : Mica naturel, fluorophlogopite, borosilicate, aluminium, et autres. Ces substrats sont ensuite recouverts soit uniquement de dioxyde de titane, ce qui correspond aux nacres blanches iridescentes, soit de différentes couches de pigments minéraux (dioxyde de titane, oxyde de fer, ferrocyanure de fer, et autres) et de pigment organiques (tels que red 7, red 34, yellow 5, et autres).

Concernant les paillettes, les substrats principaux sont le polyéthylène téréphtalate et le poly butylène téréphtalate. Les résines utilisées pour recouvrir la fine couche d'aluminium et/ou colorer la paillette sont à base de polyuréthane 11, polyuréthane 33, copolymère acryliques ou copolymère acétate de vinyle/éthylène.

Les pigments peuvent être blancs ou colorés, minéraux et/ou organiques. On peut ainsi citer, parmi les pigments minéraux le dioxyde de titane, les oxydes de zirconium ou de cérium, les oxydes de zinc, les oxydes de chrome, les différentes nuances d'oxyde de fer (noir, jaune ou rouge), le bleu d'outremer, le ferrocyanure de fer, le violet de manganèse, le bleu outremer et autres.

Parmi les pigments organiques on peut citer plus particulièrement le Red 6, le Red 7, le Red 30, le Red 34, le Yellow 5, le Bleu 1, et plus généralement les pigments insolubles de type D & C.

Les pigments nacrés peuvent se présenter sous la forme par exemple de particules de mica ou de borosilicate revêtues d'une ou plusieurs couches d'oxyde de titane et/ou d'oxyde de fer, permettant de conférer, par réflexion et réfraction de la lumière, des reflets au vernis à l'état sec.

Le pigment, ou le mélange de pigments, est de préférence présent dans la composition dans une quantité comprise entre 0.001% et 15% en poids, de préférence entre 0.005% et 12% en poids, plus préférentiellement encore entre 0.01% et 10% en poids, par rapport au poids total de la composition.

Les compositions selon l'invention peuvent éventuellement contenir, en plus des matières colorantes insolubles précitées, un ou plusieurs colorants qui seraient solubles, au moins pour partie, dans le solvant cosmétiquement acceptable mis en œuvre.

### Additifs éventuels

A côté des ingrédients essentiels précédemment définis, la composition selon l'invention peut en outre, de façon facultative, contenir des additifs complémentaires usuels dans le domaine de la cosmétique, comme par exemple des agents anti-UV, des agents anti-oxydants, des agents de surface comme des silicones, des parfums, ou encore des actifs tels que des vitamines, des protéines ou des extraits végétaux.

### Préparation des compositions selon l'invention

Les compositions selon l'invention peuvent être préparées selon des méthodes classiques bien connues de l'homme de l'art dans le domaine de la formulation des vernis à ongles. Par ailleurs, des méthodes de préparation de ces compositions sont illustrées dans les exemples donnés ci-après.

### Articles conditionnés et utilisation des compositions selon l'invention

Les compositions selon l'invention peuvent être conditionnées dans tout type de récipients ou flacons connus en soi, en verre ou en plastique par exemple, équipés à l'une de leurs extrémités d'un système d'ouverture et de fermeture amovible (tel qu'un bouchon vissable/dévissable) permettant d'en assurer l'étanchéité aux fins d'une bonne conservation.

Les bouchons peuvent être munis d'un applicateur (pinceau, spatule, embout) qui, après trempage dans la composition, permet l'application sur l'ongle de cette composition. Selon une autre variante, l'applicateur n'est pas solidarisé au bouchon et constitue un article séparé pour former un kit en deux parties de type récipient/applicateur.

Avant emploi, la viscosité de la composition conditionnée (au repos) est généralement comprise entre 1 500 et 3 000 cP (mesurée selon la méthode BROOKFIELD en mettant en œuvre un viscosimètre BROOKFIELD DVIII+ couplé à l'aiguille N°3, l'échantillon à mesurer étant placé dans un bain thermostaté à 25°C durant 12 heures, l'aiguille étant ensuite introduite dans cet échantillon et la mesure de la viscosité réalisée à une vitesse de 6 rpm).

Au moment de l'emploi, après agitation mécanique et/ou trempage de l'applicateur, la viscosité de la composition baisse pour être alors généralement comprise entre 400 et 1 500 cP (selon la mesure Brookfield définie ci-dessus, à l'exception de la vitesse qui est fixée à 60 rpm), de façon à permettre sa parfaite application sur l'ongle par l'utilisatrice.

Après application sur l'ongle, la composition retrouve progressivement sa viscosité initiale et sèche (à température ambiante et/ou par apport de chaleur pour accélérer le séchage), de façon à former au final un film de revêtement continu et durable sur l'ongle.

Si l'utilisatrice le désire, il est ensuite possible d'éliminer le film de vernis à ongle au moyen d'un dissolvant classique, comme par exemple de l'acétate d'éthyle.

### EXEMPLES

On a préparé des produits conformes à l'invention et des produits comparatifs. Les matières premières utilisées pour la préparation de ces produits étaient les suivantes :
- **Solvants** : acétate de butyle / acétate d'éthyle
- **Plastifiant** : acétyl tributyl citrate
- **Agent filmogène principal** : Nitrocellulose à 70% dans l'alcool isopropylique, commercialisée par la Société Nobel
- **Silices pyrogénées hydrophiles** : Aerosil 90, Aerosil 200 et Aerosil 380 (de surfaces spécifiques 90, 200 et 380 m²/g, respectivement) commercialisées par la Société Evonik
- **Silices pyrogénées hydrophobes** : Aerosil R972 (traitée par diméthyl dichloro silane, surface spécifique de 110m²/g), Aerosil R805 (traitée organosilane, surface spécifique de 150m²/g), Aerosil R812 (traitée organosilane, surface spécifique de 260 m²/g) et Aerosil R816 ((traitée hexadecyl silane, surface spécifique de 190 m²/g), toutes commercialisées par la Société Evonik
- **Agent anti UV** : Uvasorb 20H® filtre UV organique (benzophénone-1) commercialisé par la Société 3V
- **Résine polyester** : résine secondaire de type polyester commercialisée par la Société Lanxess sous le nom de Uniplex 670 P, cette résine comprenant dans sa chaine principale des groupements aromatiques provenant de l'anhydride trimellitique (résine vendue à 70% d'extrait sec dans l'acétate de butyle)
- **Résine epoxy** : résine secondaire de type epoxy/tosylamide commercialisée par la Société Estron sous le nom de Polytex E 75; cette résine comprenant des groupements aromatiques provenant du diglycidyl ether de bisphénol A (résine vendue à 75% d'extrait sec dans l'acétate de butyle)
- **Résine acrylique** : résine secondaire de type acrylique commercialisée par la Société DAI sous le nom de Dianal BR 115, cette résine ne comprenant aucun groupement aromatique dans sa structure (résine vendue à 100% d'extrait sec)
- **Nacre** : mica titane commercialisé sous la référence de Flamenco gold, 6-48 µm, par la Société BASF
- **Pigment Red 34 :** pigment organique commercialisé par la Société SUN Chemical

Tous les produits ont été préparés en suivant le protocole opératoire général suivant : dans un bécher de 2L, on charge les solvants puis les silices pyrogénées. On agite pour désagglomérer. On incorpore ensuite la nitrocellulose. Une fois dissoute, on incorpore la résine secondaire, le plastifiant et l'agent anti UV sous agitation. On agite 20 minutes. Le mélange obtenu est ensuite broyé dans un broyeur à bille.

Pour conduire les tests de stabilité, les matières colorantes (pigment et nacre) sont ajoutées de la façon suivante :
- on procède d'abord à un broyage préalable du pigment Red 34 dans un milieu dont la composition est la suivante (% en poids) :

| | |
|---|---|
| ▪ Acétate de butyle | 30% |
| ▪ Acétate d'éthyle | 30% |
| ▪ Plastifiant | 10% |
| ▪ Red 34 | 10% |
| ▪ Nitrocellulose | 20% |

- ensuite, pour chacun des essais décrits ci-après, on prépare les teintes finales à tester de la manière suivante (% en poids) :

| | |
|---|---|
| ▪ Composition de l'exemple concerné | 95% |
| ▪ Solution de Red 34 (telle que préparée ci-dessus) | 3% |
| ▪ Nacre | 2% |

La stabilité des produits ainsi préparés a été évaluée par simple observation visuelle après conservation d'un mois à 45°C.

Les produits identifiés par la lettre I sont conformes à l'invention, et ceux identifiés par lettre C sont des exemples comparatifs (les chiffres correspondent à des % en poids par rapport à l'ensemble de la composition).

| **Exemples N°** | **C1** | **I1** | **I2** | **C2** | **I3** | **I4** |
|---|---|---|---|---|---|---|
| Acétate de butyle | 26,95 | 26,95 | 26,95 | 26,95 | 26,95 | 26,95 |
| Acétate d'éthyle | 27,75 | 27,75 | 27,75 | 27,75 | 27,75 | 27,75 |
| Nitrocellulose | 20,25 | 20,25 | 20,25 | 20,25 | 20,25 | 20,25 |
| Anti UV | 0,2 | 0,2 | 0,2 | 0,2 | 0,2 | 0,2 |
| Plastifiant | 8 | 8 | 8 | 8 | 8 | 8 |
| Résine polyester | 15,6 | 15,6 | 15,6 | 0 | 0 | 0 |
| Résine epoxy | 0 | 0 | 0 | 15,6 | 15,6 | 15,6 |
| Aerosil 90 | 1,25 | 0 | 0 | 1,25 | 0 | 0 |
| Aerosil 200 | 0 | 1,25 | 0 | 0 | 1,25 | 0 |
| Aerosil 380 | 0 | 0 | 1,25 | 0 | 0 | 1,25 |

Après 1 mois à 45°C, les produits C1 et C2 sont instables, avec présence d'une synérèse. A l'inverse, les produits I1 à I4 sont homogènes et ne présentent pas de synérèse ni de sédimentation.

On a reproduit l'exemple I2 mais en faisant varier le taux de Aerosil 380 dans la composition :

| **Exemples N°** | **I5** | **I6** | **I7** |
|---|---|---|---|
| Acétate de butyle | 27,3 | 27,2 | 26,2 |
| Acétate d'éthyle | 27,75 | 27,75 | 27,75 |
| Nitrocellulose | 20,25 | 20,25 | 20,25 |
| Anti UV | 0,2 | 0,2 | 0,2 |
| Plastifiant | 8 | 8 | 8 |
| Résine polyester | 15,6 | 15,6 | 15,6 |
| Aerosil 380 | 0,9 | 1 | 2 |

Après 1 mois à 45°C, tous les produits I5 à I7 sont stables, homogènes et ne présentent pas de synérèse, ni de sédimentation.

| **Exemples N°** | **C3** | **C4** | **I8** | **I9** | **I10** |
|---|---|---|---|---|---|
| Acétate de butyle | 29,175 | 29,175 | 29,175 | 29,175 | 29,175 |
| Acétate d'éthyle | 26,375 | 26,375 | 26,375 | 26,375 | 26,375 |
| Nitrocellulose | 18,25 | 18,25 | 18,25 | 18,25 | 18,25 |
| Anti UV | 0,2 | 0,2 | 0,2 | 0,2 | 0,2 |
| Plastifiant | 9 | 9 | 9 | 9 | 9 |
| Résine polyester | 15 | 15 | 15 | 15 | 0 |
| Résine epoxy | 0 | 0 | 0 | 0 | 15 |
| Aerosil R972 | 2 | 0 | 0 | 0 | 0 |
| Aerosil R805 | 0 | 2 | 0 | 0 | 0 |
| Aerosil R812 | 0 | 0 | 2 | 0 | 0 |
| Aerosil R816 | 0 | 0 | 0 | 2 | 2 |

Après 1 mois à 45°C, les produits C3 et C4 sont instables, avec présence d'une synérèse. A l'inverse, les produits I8 à I10 sont homogènes, stables et ne présentent pas de synérèse ni de sédimentation.

| **Exemples N°** | **I11** | **C5** |
|---|---|---|
| Acétate de butyle | 32,9 | 36,7 |
| Acétate d'éthyle | 26,6 | 26,6 |
| Nitrocellulose | 18,25 | 18,25 |
| Anti UV | 0,2 | 0,2 |
| Plastifiant | 8 | 8 |
| Résine polyester | 12,8 | 0 |
| Résine acrylique | 0 | 9 |
| Aerosil 200 | 1,25 | 1,25 |

Sur la composition C5, on observe, après 1 mois à 45 °C, une sédimentation de ces nacres, qui s'explique par le fait qu'avec le type de résine secondaire utilisée, la silice pyrogénée n'arrive pas à exprimer de propriétés thixotropes. A l'inverse, avec la composition I11, on obtient, dans les mêmes conditions, un produit parfaitement stable et homogène.

En conclusion, tous ces exemples démontrent clairement que seules les compositions qui associent à la fois, conformément à l'invention, des silices pyrogénées (hydrophiles ou hydrophobes) de haute surface spécifique et des résines secondaires convenablement sélectionnées (présence de groupements aromatiques dans leur structure chimique) permettent d'obtenir des vernis à ongle à base de nitrocellulose présentant des propriétés améliorées notamment en terme de stabilité.

## Revendications

1. Composition pour vernis à ongles comprenant, dans un ou des solvants organiques cosmétiquement acceptables :
- un agent filmogène principal comprenant de la nitrocellulose,
- au moins une silice pyrogénée présentant une surface spécifique BET d'au moins 170 m²/g,
- au moins une résine secondaire présentant dans sa structure chimique au moins un groupement aromatique,
- au moins un plastifiant,
- au moins une matière colorante, insoluble dans ledit solvant,
ladite composition étant exempte ou substantiellement exempte de composés argileux, notamment d'argiles modifiées, en particulier d'argiles organophiles.

2. Composition selon la revendication 1, **caractérisée par le fait qu'**elle est anhydre ou substantiellement anhydre.

3. Composition selon l'une des revendications 1 à 2, **caractérisée par le fait que** le ou les solvant(s) organique(s) représente(nt) de 10% à 95%, de préférence de 30% à 90%, et plus préférentiellement encore de 50% à 85%, du poids total de la composition.

4. Composition selon l'une des revendications 1 à 3, **caractérisée par le fait que** la nitrocellulose représente de 5% à 30%, de préférence de 5% à 25%, encore plus préférentiellement de 10% à 22%, du poids total de la composition.

5. Composition selon l'une des revendications 1 à 4, **caractérisée par le fait que** la silice pyrogénée est une silice hydrophile et/ou une silice hydrophobe.

6. Composition selon l'une des revendications 1 à 5, **caractérisée par le fait que** la silice pyrogénée présente une surface spécifique BET d'au moins 190 m²/g, de préférence d'au moins 250 m²/g, et encore plus préférentiellement d'au moins 300 m²/g.

7. Composition selon l'une des revendications 1 à 6, **caractérisée par le fait que** la silice pyrogénée représente de 0.1% à 6% en poids, de préférence de 0.3% à 5%, plus préférentiellement encore de 0.5% à 4%, du poids total de la composition.

8. Composition selon l'une des revendications 1 à 7, **caractérisée par le fait que** la matière colorante est un pigment organique ou minéral.

9. Composition selon la revendication 8, **caractérisée par le fait que** le pigment représente de 0.001% à 15%, de préférence de 0.005% à 12%, plus préférentiellement encore de 0.01% à 10%, du poids total de la composition.

10. Composition selon l'une des revendications 1 à 9, **caractérisée par le fait que** la ou les résines secondaires présentant des groupements aromatiques représentent de 1% à 20%, de préférence de 2% à 20%, plus préférentiellement encore de 3% à 15%, du poids total de la composition, ces concentrations étant exprimées en extrait sec de résine.

11. Composition selon l'une des revendications 1 à 10, **caractérisée par le fait que** le ou les plastifiants représentent de 1% à 15 %, de préférence de 2% à 12%, plus préférentiellement encore de 4% à 10%, du poids total de la composition.

12. Utilisation d'une composition telle que définie à l'une quelconque des revendications 1 à 11 pour la protection et/ou le maquillage des ongles.

13. Procédé de protection et/ou de maquillage des ongles, qui comprend l'application sur lesdits ongles d'une composition telle que définie à l'une quelconque des revendications 1 à 11.

14. Article comprenant (i) un récipient muni d'un système de fermeture et rempli d'une composition telle que définie à l'une quelconque des revendications 1 à 11 et (ii) un applicateur, solidarisé ou non audit système de fermeture, et destiné, après trempage dans ledit récipient, à déposer ladite composition sur un ongle.

## Patentansprüche

1. Nagellackzusammensetzung, umfassend, in einem oder mehreren kosmetisch annehmbaren organischen Lösungsmitteln:
- Hauptfilmbilder, der Nitrocellulose umfasst,
- mindestens eine pyrogene Kieselsäure mit einer spezifischen Oberfläche BET von mindestens 170 m²/g,
- mindestens ein sekundäres Harz, das in seiner chemischen Struktur mindestens eine aromatische Gruppe aufweist,
- mindestens einen Weichmacher,
- mindestens einen Farbstoff, der in dem genannten Lösungsmittel unlöslich ist,
wobei die genannte Zusammensetzung keine oder im Wesentlichen keine tonhaltigen Verbindungen, insbesondere modifizierte Tone, insbesondere organophile Tone, aufweist.

2. Zusammensetzung nach Anspruch 1, **dadurch gekennzeichnet, dass** sie wasserfrei oder im Wesentlichen wasserfrei ist.

3. Zusammensetzung nach einem der Ansprüche 1 bis 2, **dadurch gekennzeichnet, dass** das oder die organischen Lösungsmittel von 10% bis 95%, vorzugsweise von 30% bis 90% und noch bevorzugter von 50% bis 85%, bezogen auf das Gesamtgewicht der Zusammensetzung, ausmachen.

4. Zusammensetzung nach einem der Ansprüche 1 bis 3, **dadurch gekennzeichnet, dass** die Nitrocellulose von 5% bis 30%, vorzugsweise von 5% bis 25%, noch bevorzugter von 10% bis 22%, bezogen auf das Gesamtgewicht der Zusammensetzung, ausmacht.

5. Zusammensetzung nach einem der Ansprüche 1 bis 4, **dadurch gekennzeichnet, dass** die pyrogene Kieselsäure eine hydrophile Kieselsäure und/oder eine hydrophobe Kieselsäure ist.

6. Zusammensetzung nach einem der Ansprüche 1 bis 5, **dadurch gekennzeichnet, dass** die pyrogene Kieselsäure eine spezifische Oberfläche BET von mindestens 190 m²/g, vorzugsweise mindestens 250 m²/g und noch bevorzugter mindestens 300 m²/g, aufweist.

7. Zusammensetzung nach einem der Ansprüche 1 bis 6, **dadurch gekennzeichnet, dass** die pyrogene Kieselsäure von 0,1% bis 6-Gew. %, vorzugsweise von 0,3% bis 5%, noch bevorzugter von 0,5% bis 4%, bezogen auf das Gesamtgewicht der Zusammensetzung, ausmacht.

8. Zusammensetzung nach einem der Ansprüche 1 bis 7, **dadurch gekennzeichnet, dass** der Farbstoff ein organisches oder anorganisches Pigment ist.

9. Zusammensetzung nach Anspruch 8, **dadurch gekennzeichnet, dass** das Pigment von 0,001% bis 15%, vorzugsweise von 0,005% bis 12%, noch bevorzugter von 0,01% bis 10%, bezogen auf das Gesamtgewicht der Zusammensetzung, ausmacht.

10. Zusammensetzung nach einem der Ansprüche 1 bis 9, **dadurch gekennzeichnet, dass** das oder die sekundären Harze aromatische Gruppen aufweisen, die von 1% bis 20%, vorzugsweise von 2% bis 20%, noch bevorzugter von 3% bis 15%, bezogen auf das Gesamtgewicht der Zusammensetzung, ausmachen, wobei diese Konzentrationen als Harztrockenextrakt ausgedrückt sind.

11. Zusammensetzung nach einem der Ansprüche 1 bis 10, **dadurch gekennzeichnet, dass** der oder die Weichmacher von 1% bis 15%, vorzugsweise von 2% bis 12%, noch bevorzugter von 4% bis 10%, bezogen auf das Gesamtgewicht der Zusammensetzung, ausmachen.

12. Verwendung einer Zusammensetzung, wie in einem der Ansprüche 1 bis 11 definiert, zum Schützen und/oder zum Schminken der Nägel.

13. Verfahren zum Schützen und/oder Schminken der Nägel, welches das Aufbringen einer Zusammensetzung, wie in einem der Ansprüche 1 bis 11 definiert, auf die genannten Nägel umfasst.

14. Artikel, umfassend (i) einen Behälter, versehen mit einem Verschlusssystem und gefüllt mit einer Zusammensetzung, wie in einem der Ansprüche 1 bis 11 definiert, und (ii) einen Applikator, der mit dem genannten Verschlusssystem fest verbunden oder nicht fest verbunden ist und dazu bestimmt ist, nach dem Eintauchen in den genannten Behälter, die genannte Zusammensetzung auf einen Nagel aufzutragen.

## Claims

1. Nail varnish composition comprising, in one or more cosmetically acceptable organic solvents:
- a main film-forming agent comprising nitrocellulose,
- at least one pyrogenic silica having a BET specific surface area of at least 170 m²/g,
- at least one secondary resin having in its chemical structure at least one aromatic group,
- at least one plasticizer,
- at least one coloring material, insoluble in said solvent,
said composition being free or substantially free of clay compounds, in particular modified clays, in particular organophilic clays.

2. Composition according to claim 1, **characterized by** the fact that it is anhydrous or substantially anhydrous.

3. Composition according to one of claims 1 to 2, **characterized by** the fact that the organic solvent(s) represent(s) from 10% to 95%, preferably from 30% to 90%, and more preferentially still from 50% to 85%, of the total weight of the composition.

4. Composition according to one of claims 1 to 3, **characterized by** the fact that the nitrocellulose represents from 5% to 30%, preferably from 5% to 25%, even more preferentially from 10% to 22%, of the total weight of the composition.

5. Composition according to one of claims 1 to 4, **characterized by** the fact that the pyrogenic silica is a hydrophilic silica and/or a hydrophobic silica.

6. Composition according to one of claims 1 to 5, **characterized by** the fact that the pyrogenic silica has a BET specific surface area of at least 190 m²/g, preferably at least 250 m²/g, and even more preferentially at least 300 m²/g.

7. Composition according to one of claims 1 to 6, **characterized by** the fact that the pyrogenic silica represents from 0.1 % to 6% by weight, preferably from 0.3% to 5%, more preferentially still from 0.5% to 4%, of the total weight of the composition.

8. Composition according to one of claims 1 to 7, **characterized by** the fact that the coloring material is an organic or inorganic pigment.

9. Composition according to claim 8, **characterized by** the fact that the pigment represents from 0.001% to 15%, preferably from 0.005% to 12%, more preferentially still from 0.01% to 10%, of the total weight of the composition.

10. Composition according to one of claims 1 to 9, **characterized by** the fact that the secondary resin(s) having aromatic groups represent(s) from 1% to 20%, preferably from 2% to 20%, more preferentially still from 3% to 15%, of the total weight of the composition, these concentrations being expressed as resin dry extract.

11. Composition according to one of claims 1 to 10, **characterized by** the fact that the plasticizer(s) represent(s) from 1% to 15%, preferably from 2% to 12%, more preferentially still from 4% to 10%, of the total weight of the composition.

12. Use of a composition as defined in any one of claims 1 to 11 for the protection and/or make-up of the nails.

13. Process for protecting and/or making-up the nails, which comprises the application to said nails of a composition as defined in any one of claims 1 to 11.

14. Article comprising (i) a container provided with a closure system and filled with a composition as defined in any one of claims 1 to 11 and (ii) an applicator, optionally integral with said closure system, intended, after dipping into said container, for depositing said composition on a nail.
